# EUROPEAN PATENT APPLICATION

(11) **EP 1 964 588 A1**
(43) Date of publication of application: **03.09.2008**
(21) Application number: 08356033.4
(22) Date of filing: 03.03.2008
(51) Int. Cl.: A61M 5/32

(54) **Shield for covering the extremity of an administration device or assembly, an administration assembly and an administration device**

(30) Priority: 02.03.2007 FR 0701532
(71) Applicant: Becton Dickinson France, 38800 Le Pont-de-Claix (FR)
(72) Inventor: Felix-Faure, Catherine, 38000 Grenoble (FR)
(74) Representative: Brédeville, Odile Marie

(57) **Abstract**

The invention relates to a shield (1) for covering at least part of a needle (2) and needle hub (6) of a needle assembly, said shield (1) having an open proximal end (1a), a closed distal end (1b) and a longitudinal wall (4) extending from said proximal end (1a) to said distal end (1b) and defining an interior cavity (5) for receiving at least part of the needle (2) and needle hub (6), characterized in that it comprises at least a first retainer (9) defined on said wall (4) and extending into said cavity (5) and, at least one second retainer (8) defined on said wall (4) and extending into said cavity (5), said annular bead portion (9) and second retainer (8) being designed to be able to, resiliently and releasably, engage a part of said needle assembly to secure said shield (3) thereto, said first retainer (9) and second retainer (8) having at least their shape or one of their dimensions different from the other.

The invention also relates to a needle assembly and to an injection device, each comprising such a shield.

## Description

The present invention relates to a shield for covering the extremity of an administration device or of an assembly at least prior to use of the administration device or assembly.

In this application, the term distal means the part furthest from the user's hand, and the term proximal means the part closest to the user's hand. Likewise, in this application, the term "distal direction" means the direction of administration, i.e., towards the patient, and the term "proximal direction" means direction opposite to the direction of administration, i.e., away from the patient.

Aministration devices are commonly used in several technical fields such as, for example, the medical field to administer to a patient, for example a medical product, either by spraying for spraying devices or by injection for injection devices. To do so, the extremity of the administration device can be provided with a staked needle or nozzle or a luer connection allowing provision of an assembly such as a nozzle assembly or a needle assembly.

In the present application, by "staked needle" one means a needle fixed on the tip of the injection device by gluing or by any other suitable method such as for example shrinking of the tip of the syringe surrounding the needle after heating.

In the medical field, injection devices, such as syringes provided with needles, either staked needles or needle assemblies on luer syringes, are usually provided to the end-users with needle shields: indeed, needles must remain sterilized until use and need to be protected from possible contamination from the environment. Similarly, injection device such as syringe with no needle, provided luer or with nozzle or with luer lock fitting, are usually provided to the end-users with cap protecting the extremity of the luer or of the nozzle from possible contamination from the environment. Moreover, in the case of injection devices with needle, the end user must also be protected from accidental needle-stick injuries.

These needle shields or caps are usually made of an elastic material, such as a thermoplastic elastomer, and are assembled on the syringe extremity for instance on assembly lines of industrial pharmaceutical companies. In the case of syringe with staked needle, the distal end of the syringe usually comprises a bulging part or hub to which the needle is secured by gluing and upon which the needle shield is removably engaged by friction, for example. One of the problems encountered during such an assembly step of the needle shield on the hub is the generation or the revealing of particles caused by the friction between the needle shield and the bulging part of the syringe hub that bears the needle. This causes important quality issues.

This problem may also occur when the needle shield is withdrawn from the syringe hub.

In this application, the term "revealing" means that some particles may already exist or be preformed in the needle shield and are displaced and/or broken away during the assembling or removing of the needle shield from the syringe hub.

Similar problems may occur with syringes provided luer or with nozzles covered by caps.

Moreover, similar problems may occur with assemblies such as nozzle assembly or needle assembly and provided to be connected to a luer spraying or injection device.

In addition to the previous problem described, once the syringe or any other administration device is delivered to the end-user, the needle shield must be removed from the syringe hub in order to free the needle before the syringe is ready for an injection. This operation may be delicate, since the end user must take care not to remove the needle shield too quickly in order to avoid aspiration, by the needle shield and through the needle, of the liquid product contained in the syringe. At the same time, because the needle shield must sealingly close the syringe hub for reasons of sterility, the needle shield is tightly fixed to the syringe hub and its removal may be quite difficult. As previously mentioned, similar problem may occur with other administration devices provided luer or with spraying or injection assemblies.

US 6,719,732 B2 discloses a protection device for a syringe needle comprising an elastic needle shield of general longitudinal direction presenting a closed distal end and an open proximal end, the needle shield being formed by a lateral wall defining an inner housing intended to receive the hub of a syringe body. A generally continuous annular bead is provided on the lateral wall and disposed in the housing and includes at least one slot extending longitudinally through the annular bead. The slot permits sterilizing gas to pass into the housing without causing the needle shield to detach from the syringe during the sterilization process. The annular bead provides for generally continuous contact between the needle shield and the syringe - except for the slot. Therefore, the needle shield disclosed in this patent is held in place on the syringe in a securely way. Nevertheless, the assembling of the needle shield on the syringe and its withdrawal require to exert forces that can be felt to be too important by the users. In addition, theses forces may lead to particules generation or revealing linked with the high level of friction between the annular bead and the syringe.

There is therefore a need for a protection device that would be securely attached to an administration device or of an assembly to safely and efficiently protect the extremity of the administration device or assembly and would be, at the same time:
- on a process level easy to assemble on the tip of an administration device such as a syringe hub or on an administration assembly, and
- on an end-user level easy to disassemble,
and in both cases that would limit the generation or revealing of particles. The present invention overcomes the above-described shortcomings of the prior.

In accordance with an embodiment of the present invention, there is proposed a shield for covering at least part of the extremity of an administration device or of an assembly, said shield having an open proximal end, a closed distal end and a longitudinal wall extending from said proximal end to said distal end and defining an interior cavity for receiving at least part of the extremity of the administration device or of the assembly, characterized in that it comprises at least a first retainer defined on said wall and extending into said cavity and, at least one second retainer defined on said wall and extending into said cavity, said first retainer and second retainer being designed to be able to, resiliently and releasably, engage a part of said extremity of the administration device or assembly to secure said shield thereto, said first retainer having at least its shape or one of its dimensions different from the shape or dimensions of said second retainer, said first retainer and second retainer resiliently and releasably engaging the same part of said extremity of the administration device or assembly.

The combination of the first and second retainers permits to limit the friction surface between the administration device or assembly and the shield, therefore, the generation or revealing of particles. In addition, the second retainer can act as a lever to amplify and ease the deformation of the first retainer during the assembling and/or the withdrawing step of the shield on or from the administration device or assembly.

In an embodiment of the invention, said first retainer and said second retainer are located in the same transversal plane.

In an embodiment of the invention, said first retainer comprises at least a bead portion that extends radially into said interior cavity on a first predetermined distance Db, and said second retainer comprises at least a projection that extends radially into said interior cavity on a second predetermined distance Dp that is different than said first predetermined distance Db. In particular, when Dp is greater than Db, it allows the second retainer to act as a lever in regards to the first retainer.

In an embodiment of the invention, said first retainer extends longitudinally into said interior cavity on a first predetermined length Hb, and said second retainer extends longitudinally into said interior cavity on a second predetermined length Hp that is different than said first predetermined length Hb. In particular, when Hp is greater than Hb, it allows an easier removal and facilitated assembly of the shield on the administration device or assembly.

In an embodiment of the invention, said first retainer or second retainer comprises a continuous annular bead.

In an embodiment of the invention, the shield comprises at least two first retainers and/or second retainers.

In an embodiment of the invention, each of the said two first retainers comprises a bead portion and/or each of the said two second retainers comprises a projection, said bead portions and said projections being located in the same transversal planes and regularly spaced from each other.

In another embodiment, said each of the said two first retainers comprises a bead portion and/or each of the said two second retainers comprises a projection, said bead portions and said projections being located in different transversal planes.

In another embodiment of the invention, at least two of said first retainers and/or second retainers are located in different transversal planes.

In an embodiment of the invention, at least one of said first retainer or said second retainer has a proximal face forming a predetermined angle β with the longitudinal axis of said shield. Preferably, said angle β ranges from 35° to 60°.

In an embodiment of the invention, at least one of said first retainer or said second retainer has a distal face forming a predetermined angle α with the longitudinal axis of said shield. Preferably, said angle α ranges from 20° to 40°.

Because of the specific angular arrangement of the proximal face of said first retainer and/or said second retainer, the removal of the shield from the distal part of an administration device or assembly by the end user is facilitated. In particular, the angular arrangement creates a smooth slope that allows the end user to retrieve the shield slowly without having to pull too greatly on the shield: the risk of aspiration of liquid and/or of accidental needle stick injury due to a too sharp movement is therefore prevented. In addition the slope formed by the specific angular arrangement of the distal face of the annular bead and/or projection facilitates the assembly of the needle shield on the extremity of an administration device or of an assembly before it is provided to the end user, hence reducing particles generation and/or revealing.

In an embodiment of the invention, at least part of the shield is made from an elastically deformable material and is contained within a shell made at least in part from a rigid material. The shield is therefore protected from the risk of external shocks, more easily handled for assembling step and shield removal. In addition, when the administration device is an injection device provided with a needle, or when the assembly comprises a needle, the rigid material prevents the needle from piercing the shield when, after use, the user put the shield back over the needle.

In an embodiment of the invention, at least one of said first retainer and/or said second retainer have a cross-sectional shape of half drop of water of which widest part faces the distal end of said shield. This specific shape improves the reliability of the positioning and the holding of the shield on the administration device or on the assembly and enable the removal of the shield with a reduced force.

In an embodiment of the invention, said first retainer and/or said second retainer define for said cavity a predetermined geometric shape. Said predetermined shape may be chosen in the group comprising a square, a triangle, an oblong format, a cross, a star.

In an embodiment of the invention, the shield further comprises an outer casing formed of a first material and an inner casing formed of a second material different from the said first material, said inner casing defining said interior cavity for receiving, in an impermeable way, at least part of the extremity of the administration device or of the assembly, and aspiration limiting means for limiting the deformation of said interior cavity when said shield is separated from the assembly.

In an embodiment of the invention, the aspiration limiting means may comprise bearing surfaces designed for being in contact with said inner casing and for cooperating with said inner casing in order to limit the stretching of said inner casing when said shield is separated from said assembly.

Another aspect of the invention is an assembly for an administration device, said assembly comprising at least an assembly hub for assembling said assembly on said administration device, characterized in that it further comprises a shield as described above.

Another aspect of the invention is an administration device comprising at least a container and an extremity, characterized in that it further comprises a shield as decribed above provided on said extremity.

Other advantages of the present invention will now be specified with the aid of the description which follows and of the attached drawings in which:
Figure 1 is a cross section view of an embodiment of the shield of the invention,
Figure 2 is a perspective view of the shield of figure 1,
Figure 3 is a cross section view of the shield of figure 1 along transversal plane P1,
Figure 4 is a cross section view of the shield of figure 1 with a syringe hub ready to be assembled on the shield,
Figure 5 is a cross section view of the shield of figure 4 once assembled on the syringe hub,
Figures 6A, 6B and 6C are respectively a perspective view, a cross section view according to the longitudinal axis of a perspective view and half of a bottom view of an alternative of the shield of the invention,
Figures 7A, 7B and 7C are respectively a perspective view, a cross section view according to the longitudinal axis and half of a bottom view of an alternative of the shield of the invention,
Figures 8A, 8B and 8C are respectively a perspective view, a cross section view according to the longitudinal axis and half of a bottom view of an alternative of the shield of the invention,
Figure 9 is a cross section view of another alternative embodiment of the shield according to the invention contained in a shell,
Figures 10A to 10H are cross section views similar to figure 3 for alternative embodiments of the shield according to the invention,
Figure 11 is a partial cross section view of an alternative embodiment of the shield according to the invention of figure 1, and
Figure 12 is a cross section view of the inner casing of the shield of figure 11 along transversal plane P1.

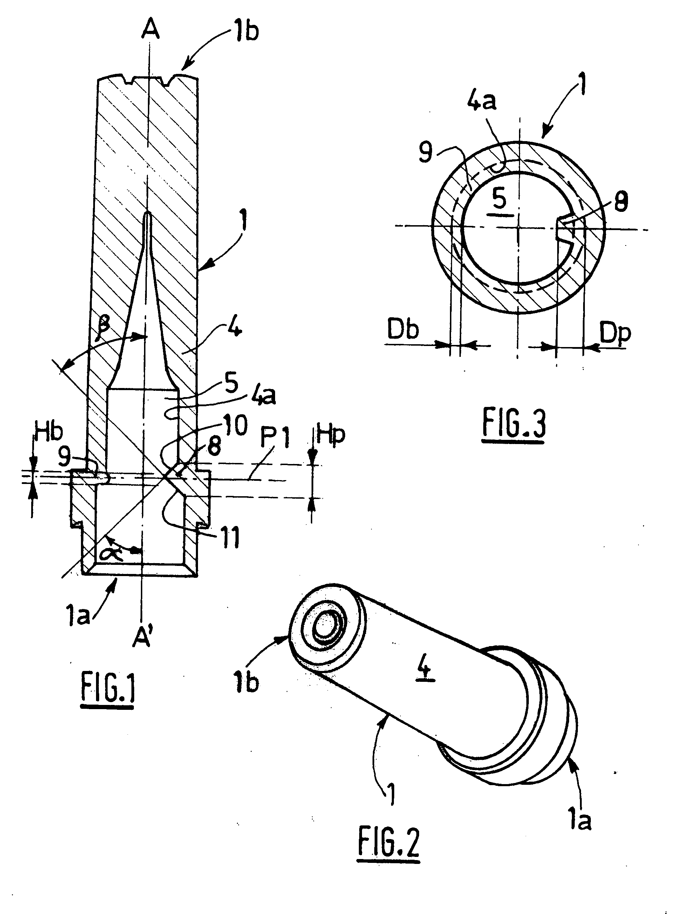

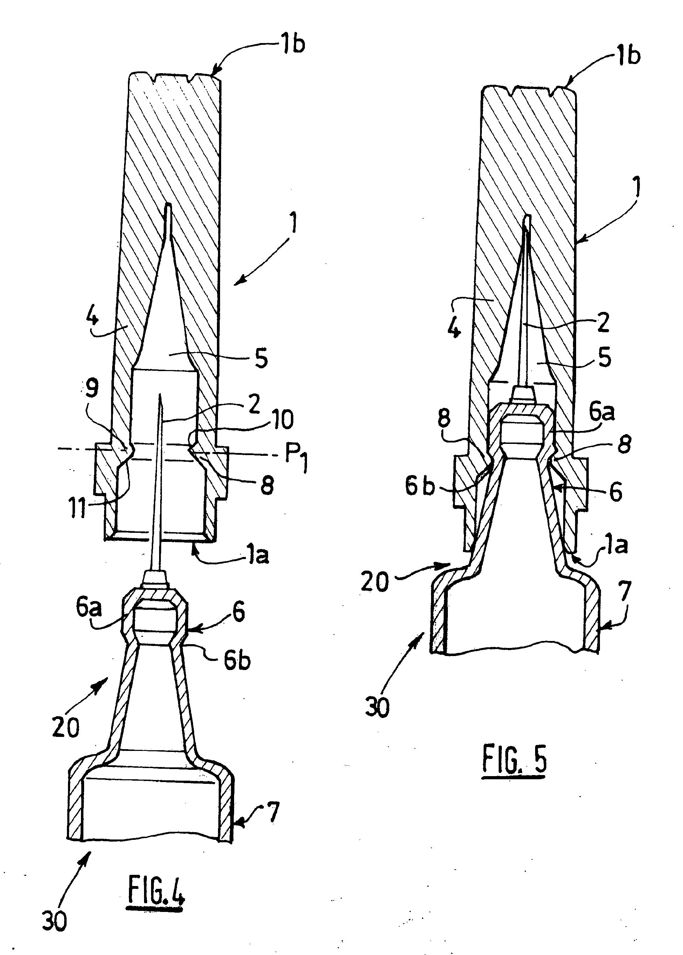

On Figures 1 and 2, is shown a shield 1 according to the invention having the general shape of cap, with an open proximal end 1 a, a closed distal end 1b and a longitudinal wall 4 extending from said proximal end 1 a to said distal end 1b and defining an interior cavity 5. This cavity 5 is intended to receive at least part of a needle and needle hub (both shown on figures 4 and 5). The shield 1 of figure 1 comprises a first retainer forming an annular bead portion 9 defined on the wall 4 and extending from the internal face 4a of said wall 4 into the cavity 5. The annular bead portion 9 is continuous and forms a continuous annular bead around the whole said cavity 5 (see figure 3).

The shield 1 also comprises a second retainer forming a projection 8 extending from the annular bead portion 9 into the cavity 5. As is shown on figure 1, the annular bead 9 and the projection 8 are located in the same transversal plane P1.

Figure 3 is a cross section view of the shield 1 of figure 1 along the transversal plane P1 of figure 1: on figure 3 the internal face 4a of the wall 4 is shown in dashes. As appears clearly on this figure, the annular bead portion 9 extends radially from said internal face 4a into said cavity 5 on a first predetermined distance Db, and said projection 8 extends radially from said internal face 4a into said interior cavity 5 on a second predetermined distance Dp that is greater than said first predetermined distance Db.

Moreover, as appears from figure 1, the annular bead portion 9 extends longitudinally into said cavity 5 on a first predetermined length Hb, and said projection 8 extends longitudinally into said cavity 5 on a second predetermined length Hp that is greater than said first predetermined length Hb.

As shown on figure 1, the projection 8 has a distal face 10 that forms an angle α with the longitudinal axis AA' of the shield 1, and a proximal face 11 that forms an angle β with said longitudinal axis AA'. The angle α preferably ranges from 20 to 40°. On the example shown, the angle α has a value of 30°. The angle β preferably ranges from 35 to 60°. On the example shown, the angle β has a value of 45°.

The shield 1 of figure 1 is made of an elastically deformable material, for instance a thermoplastic elastomer. The annular bead 9 and the projection 8 are also made of an elastically deformable material, which may be identical or different from the material forming the shield 1. For example, the annular bead 9 and the projection 8 of the shield 1 of figure 1 are made of thermoplastic elastomer.

In alternate embodiments of the invention, the shield 1 comprises one or more additional annular beads and/or projections, in addition to the first annular bead 9 and to the first projection 8 as described in reference with figure 1. The additional annular beads and projections may be identical or different in shape or in dimension from said first annular bead 9 and first projection 8 respectively. They may be located in the same transversal plane as the first annular bead 9 and/or projection 8 or on the contrary in different transversal planes.

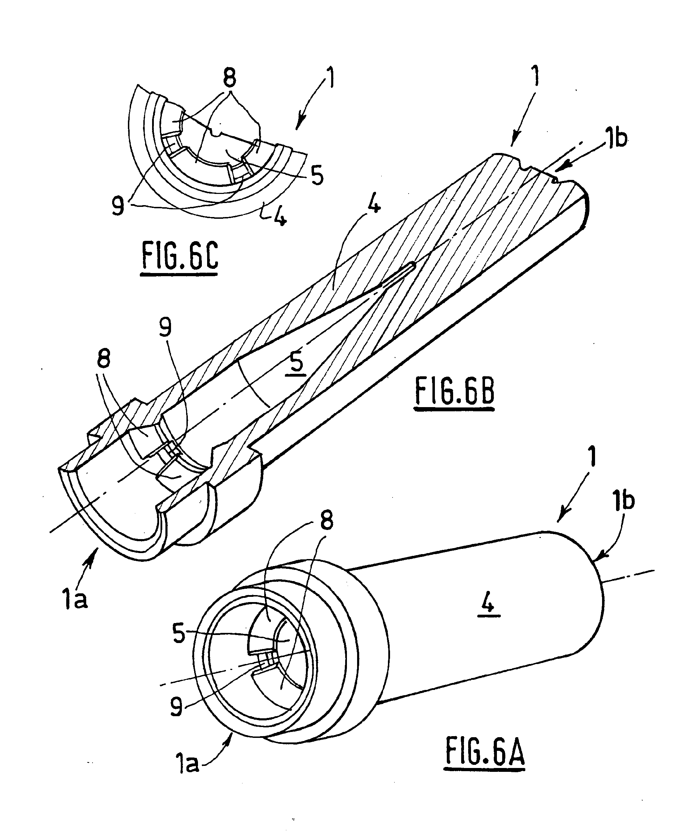

Figures 6A to 8C are illustrative views of such alternative embodiments comprising additional projections. The references designating the same elements as in figure 1 have been maintained on all these figures.

On figures 6A to 6C is shown an alternative embodiment of the shield 1 of figure 1 comprising a plurality of projections 8 regularly spaced from each other. In the example shown, the shield 1 comprises one annular bead 9 and four (partially shown) projections 8, located in the same transversal plane as the annular bead 9.

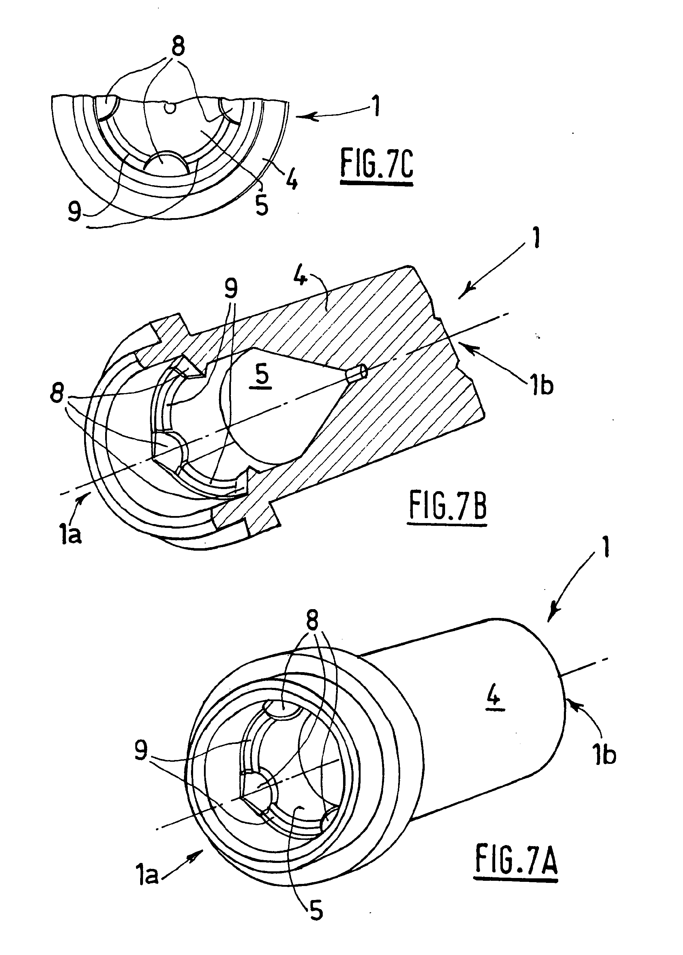

On figures 7A to 7C is shown an alternative embodiment of the shield 1 of figure 1 comprising a plurality of projections 8 regularly spaced from each other. In the example shown, the shield 1 comprises one annular bead 9 and four (partially shown) projections 8, located in the same transversal plane as the annular bead 9. The projections 8 of the shield 1 of figures 7A to 7C have the shape of half a drop of water, the widest part of said drop of water facing the distal end 1 b of the shield 1.

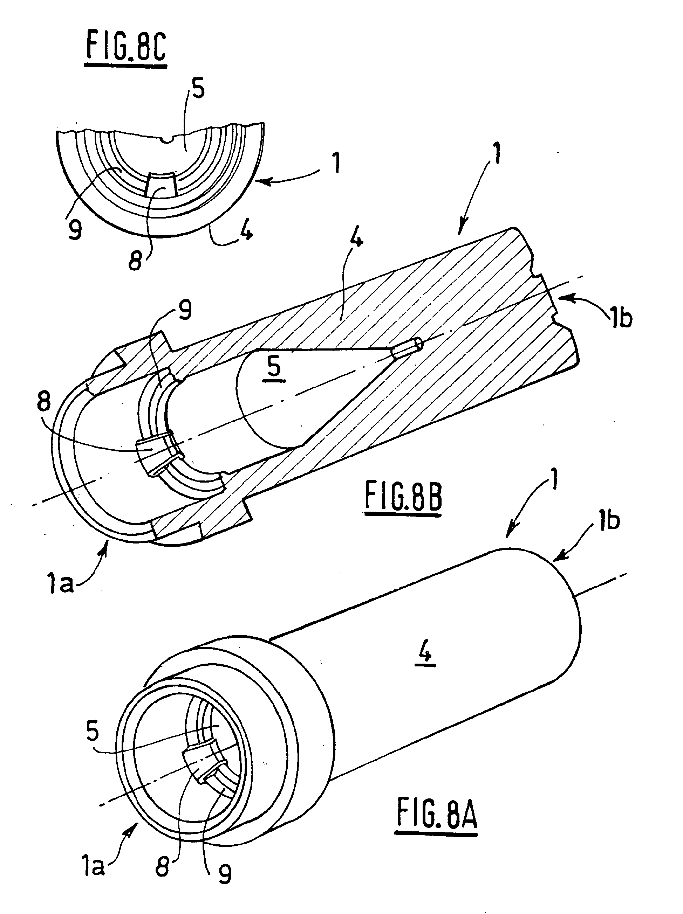

On figures 8A to 8C is shown an alternative embodiment of the shield 1 of figure 1 comprising at least one projection 8 and at least one annular bead 9.

Depending on the respective shape and number of annular beads and/or projections, said annular beads and/or projections may define for the cavity 5 a shape chosen in the group comprising a square, a triangle, an oblong format, a cross, a star.

As will appear now from the description of figures 4 and 5, the first annular bead 9, the first projection 8 and the optionally additional annular beads and projections are designed to be able to, resiliently and releasably, engage the same part of an assembly to secure said shield 1 thereto.

On figure 4 are shown the shield 1 of figure 1 and an administration device 30 (partially shown) comprising an assembly 20 and a needle hub 6 located at the distal end of a container 7, partially shown, of said administration device 30. The needle hub 6 comprises a distal end forming a bulging part 6a, which is separated from the container 7 by a portion of reduced diameter 6b of the needle hub 6. A needle 2 is fixed at the distal end of the bulging part 6a.

As appears on figure 4, the diameter of the bulging part 6a is slightly larger than the diameter of the cavity 5 in the transversal plane P1.

In consequence, during the process of the shield 1 assembly on the needle hub 6 in order to protect the needle 2, the annular bead 9 and the projection 8 deform under the pressure exerted by the bulging part 6a. Once the shield 1 is in place, as shown on figure 5, the annular bead 9 and the projection 8 come back in their rest state and are in contact and engaged with the same part of the assembly, that is to say the portion of reduced diameter 6b of the needle hub 6. The shield 1 is therefore securely fixed to the needle hub 6.

The combination of the annular bead 9 and of the projection 8 allows limiting the friction surface between the bulging part 6a of the needle hub 6 and the shield 1. The generation of particles is therefore limited. In particular, because the proximal face 11 of the projection 8 form an angle of 45° with the longitudinal axis AA' of the shield, the assembly of the shield on the needle hub 6 is smooth and eased. In particular, because Dp is greater than Db (see figure 3) the projection 8 acts as a lever to amplify and ease the deformation of the annular bead 9 during the assembly of the shield 1.

For the same reason, the removal of the shield 1 from the needle hub 6 is facilitated and does not require an excessive force. In addition, the fact that the distal face 10 of the projection 8 forms an angle of 30° with the longitudinal axis AA' of the shield 1 facilitates the removal of the shield 1 from the needle hub 6. When the user removes the shield 1 from the needle hub 6, the annular bead 9 and the projection 8 deform under the stress exerted by the bulging part 6a and then return to their normal state thanks to their inherent resilience. In this step of removal of the shield 1, the projection 8 also acts as a lever to amplify and ease the deformation of the annular bead 9.

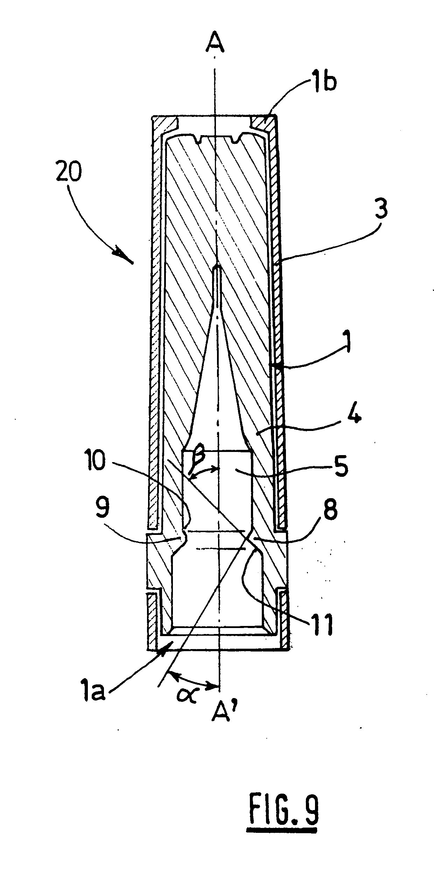

On figure 9 is shown the shield of figure 1 contained in a shell 3. The shell 3 is made of a rigid material, such as polypropylene. The shell 3 is intended to protect the shield 1 from external shocks, ease the shield 1 handling and prevent the needle from piercing the shield 1.

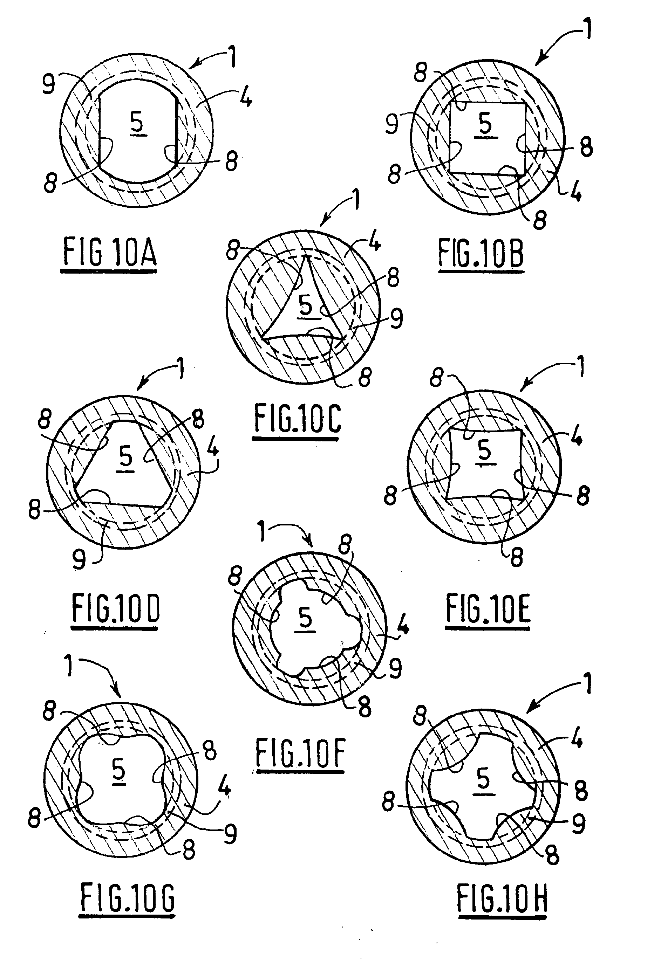

On figures 10A to 10H are shown cross section views similar to figure 3 for alternative embodiments of the shield 1 of the invention where the projections define predetermined geometric shape. In particular the projections 8 define for the cavity 5:
- in figure 10A, a shape of an oblong format,
- in figures 10B, 10E, and 10G, a shape similar to or substantially similar to a square,
- in figures 10C and 10D, a shape substantially similar to a triangle,
- in figure 10F, a shape substantially similar to a star,
- in figure 10H, a shape substantially similar to a cross.

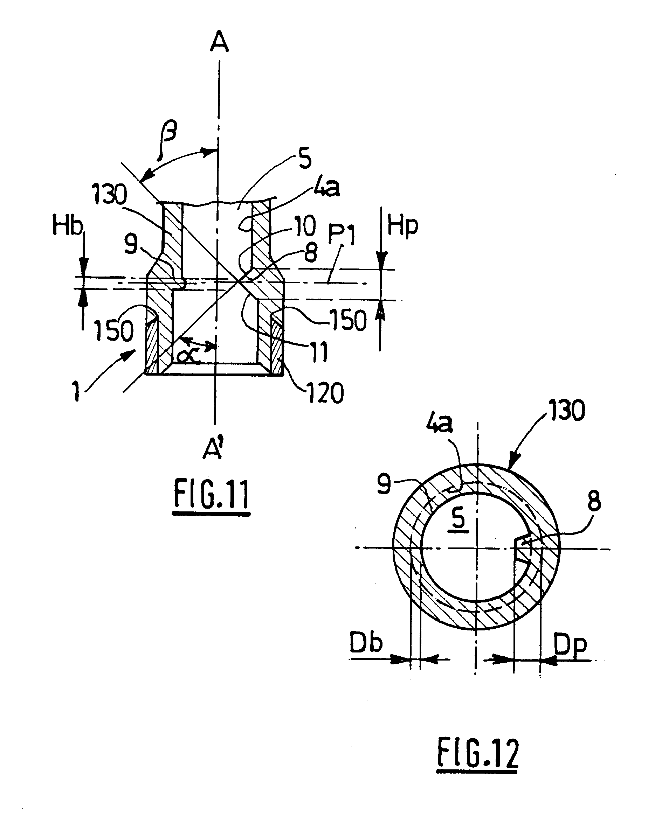

Figure 11 is a cross section view of an alternative embodiment of the shield 1 of figure 1 further comprising an outer casing 120 formed of a first material, and an inner casing 130 formed of a second material different from the said first material. The said first material and the said second material may show a different stiffness. The references designating the same elements as in figure 1 have been maintained in figure 11. In the example shown on figure 11, the inner casing 130 defines the interior cavity 5. The shield 1 of figure 11 further comprises a hook 150 formed on the outer casing 120. The hook 150 forms a bearing surface in contact with the inner casing 130. When the shield 1 is removed from the assembly (not shown), the hook 150 cooperates with the inner casing 130 so as to limit the stretching of the inner casing, in particular in regards to the outer casing 120 which is formed of a material different from the material forming the inner casing 130.

Indeed, when the shield 1 is removed from the assembly, a negative pressure may be created in the interior cavity 5, said negative pressure being likely to generate within said interior cavity the aspiration of the substance contained in the assembly. By limiting the stretching of the inner casing 130, the hook 150 forms an aspiration limiting means of the such created aspiration.

Figure 12 is a cross section view of the inner casing 130 of the shield 1 of figure 11 along the transversal plane P1 of figure 11 : on figure 12, the internal face 4a of wall 4 is shown in dashes. As is clear from this figure, the bead portion 9 extends radially from said internal face 4a into said cavity 5 on a first predetermined distance Db, and said projection 8 extends radially from said internal face 4a into said interior cavity 5 on a second predetermined distance Dp that is greater than said first predetermined distance Db.

The shield of the invention is an improvement of the needle shields of the prior art. Thanks to the shield of the invention, it is possible to efficiently protect a needle with a shield that may be assembled and/or removed on or from a syringe hub in a facilitated way and without generation of particles. Similarly, the shield of the invention can be efficiently used to protect the extremity of any kind of administration devices such as for example spraying devices and/or any kind of administration assemblies such as, for example, needle assembly, nozzle assembly.

## Claims

1. A shield (1) for covering at least part of the extremity (6) of an administration device (30) or of an assembly (20), said shield (1) having an open proximal end (1a), a closed distal end (1b) and a longitudinal wall (4) extending from said proximal end (1a) to said distal end (1b) and defining an interior cavity (5) for receiving at least part of the extremity (6) of the administration device (30) or of the assembly (20), **characterized in that** it comprises at least a first retainer (9) defined on said wall (4) and extending into said cavity (5) and, at least one second retainer (8) defined on said wall (4) and extending into said cavity (5), said first retainer (9) and second retainer (8) being designed to be able to, resiliently and releasably, engage a part of said said extremity of the administration device (30) or assembly (20) to secure said shield (1) thereto, said first retainer (9) having at least its shape or one of its dimensions different from the shape or dimensions of said second retainer (8), said first retainer (9) and second retainer (8) resiliently and releasably engaging the same part (6b) of said extremity of the administration device (30) or assembly (20).

2. A shield (1) according to claim 1, **characterized in that** said first retainer (9) and said second retainer (8) are located in the same transversal plane.

3. A shield (1) according to claim 1 or 2, **characterized in that** said first retainer comprises at least a bead portion (9) that extends radially into said interior cavity (5) on a first predetermined distance Db, and said second retainer comprises at least a projection (8) that extends radially into said interior cavity (5) on a second predetermined distance Dp that is different than said first predetermined distance Db.

4. A shield (1) according to one of claims 1 to 3, **characterized in that** said first retainer extends longitudinally into said interior cavity (5) on a first predetermined length Hb, and said second retainer extends longitudinally into said interior cavity (5) on a second predetermined length Hp that is different than said first predetermined length Hb.

5. A shield (1) according to one of claims 1 to 4, **characterized in that** said first retainer or second retainer comprises a continuous annular bead (9).

6. A shield (1) according to one of claims 1 to 5, **characterized in that** it comprises at least two first retainers (9) and/or two second retainers (8).

7. A shield (1) according to claim 6, **characterized in that** each of the said two first retainers comprises a bead portion (9) and/or each of the said two second retainers comprises a projection (8), said bead portions (9) and said projections (8) being located in the same transversal planes and regularly spaced from each other.

8. A shield (1) according to claim 6, **characterized in that** said each of the said two first retainers comprises a bead portion (9) and/or each of the said two second retainers comprises a projection (8), said bead portions (9) and said projections (8) being located in different transversal planes.

9. A shield (1) according to one of claims 1 to 8, **characterized in that** at least one of said first retainer (9) or said second retainer (8) has a proximal face (11) forming a predetermined angle β with the longitudinal axis of said shield (1).

10. A shield (1) according to claim 9, **characterized in that** said angle β ranges from 35° to 60°.

11. A shield (1) according to one of claims 1 to 10, **characterized in that** at least one of said first retainer (9) or said second retainer (8) has a distal face (10) forming a predetermined angle α with the longitudinal axis of said shield (1).

12. A shield (1) according to claim 11, **characterized in that** said angle α ranges from 20° to 40°.

13. A shield (1) according to one of claims 1 to 12, **characterized in that** at least part of said shield (1) is made from an elastically deformable material and is contained within a shell (3) made at least in part from a rigid material.

14. A shield (1) according to one of claims 1 to 13, **characterized in that** at least one of said first retainer (9) and/or said second retainer (8) have a cross-sectional shape of half drop of water of which widest part faces the distal end (1b) of said shield (1).

15. A shield (1) according to one of claims 1 to 14, **characterized in that** said first retainer and/or second retainer (8, 9) define for said cavity (5) a predetermined geometric shape.

16. A shield (1) according to claim 15, **characterized in that** said predetermined geometric shape is chosen in the group comprising a square, a triangle, an oblong format, a cross, a star.

17. A shield (1) according to one of claims 1 to 16, **characterized in that** it further comprises:
an outer casing (120) formed of a first material;
an inner casing (130) formed of a second material different from the said first material, said inner casing (130) defining said interior cavity (5) for receiving, in an impermeable way, at least part of the extremity of the administration device (2) or of the assembly, and
aspiration limiting means (150) designed for limiting the deformation of said interior cavity (5) when said shield (1) is separated from the assembly (20).

18. A shield (1) according to claim 17, **characterized in that** said aspiration limiting means comprise bearing surfaces (150) designed for being in contact with said inner casing (130) and for cooperating with said inner casing (130) in order to limit the stretching of said inner casing (130) when said shield (1) is separated from the assembly (20).

19. An assembly (20) for an administration device (30), said assembly (20) comprising at least an assembly hub for assembling said assembly on said administration device, **characterized in that** it further comprises a shield (1) according to at least claim 1.

20. An administration device (30) comprising at least a container (7) and an extremity, **characterized in that** it further comprises a shield (1) according to at least claim 1 provided on said extremity.
